Europäisches Patentamt

European Patent Office

Office européen des brevets

(11) Publication number: **0 121 980**
**B1**

(12) **EUROPEAN PATENT SPECIFICATION**

(45) Date of publication of patent specification: **13.09.89**

(21) Application number: **84300587.7**

(22) Date of filing: **30.01.84**

(51) Int. Cl.⁴: **A 61 L 2/04**

(54) Apparatus for sterilizing devices.

(30) Priority: **14.03.83 JP 40818/83**
**21.04.83 JP 58797/83**
**30.08.83 JP 159951/83**

(43) Date of publication of application:
**17.10.84 Bulletin 84/42**

(45) Publication of the grant of the patent:
**13.09.89 Bulletin 89/37**

(84) Designated Contracting States:
**DE FR GB SE**

(56) References cited:
**EP-A-0 116 921**
**WO-A-82/04188**
**FR-A-2 305 101**
**FR-A-2 493 682**
**GB-A- 390 131**
**GB-A- 515 195**
**GB-A-1 041 111**
**GB-A-1 406 789**
**GB-A-1 441 701**
**US-A-2 550 584**

(73) Proprietor: **Japan Medical Supply Company**
**Limited**
**12-17 Kako-Machi Naka-ku**
**Hiroshima City Hiroshima Pref. (JP)**

(72) Inventor: **Kato, Yasuaki**
**13-2, Omiya-cho 1-chome Nishi-ku**
**Hiroshima City Hiroshima Pref. (JP)**

(74) Representative: **Freed, Arthur Woolf**
**Reginald W. Barker & Co. 13 Charterhouse**
**Square**
**London EC1M 6BA (GB)**

Courier Press, Leamington Spa, England.

## EP 0 121 980 B1

**Description**

The present invention relates to an apparatus for sterilizing devices as defined in the first part of Claim 1.

Medical devices containing medical materials are required to be in an aseptic condition when used, and each device is sterilized before use. The sterilization treatment is made by methods using a radiation, liquid chemicals, heat or the like. In such methods, the method using the radiation and ethylene oxide gas are suitable for sterilizing devices of a disposable type and are widely used. However, such methods are not suitable for sterilizing devices to be repeatedly used, require large equipments, and are not suitable for a personal use.

Heretofore, medical devices to be repeatedly used and required to be sterilized each time they are used have been generally disinfected using liquid chemicals.

Further, a medical treatment by the peritoneal dialysis, for example, which is remarkably given attention recently requires a simple device as compared with an artificial kidney. The expense for the treatment is extremely economical and a patient's charge is greatly reduced. A continuous ambulatory peritoneal dialysis method is put into practice, and consequently the patient can even work while continuously receiving the treatment. The reliability of this dialysis method is dependent on the prevention of bacteria from entering into a dialysis tube and the sterilization treatment of a connector which connects between the dialysis tube attached to the abdominal region and a disposable container containing the dialysate. In other words, the peritoneal dialysis method utilizes the dialysis tube attached to the abdominal region which is connected to the disposable container containing the dialysate so that the dialysate is injected into the abdominal cavity from the container through the tube to make the dialysis through the peritoneum between capillaries in the peritoneum and the abdominal cavity. The dialysate is exchanged about three times a day. Each time the dialysate is exchanged, the container containing the old dialysate is removed from the tube attached to the abdominal region to exchange it for a new container. At this time, since the connector portion of the tube is exposed to the outside air, there is a possibility of contaminating the connector portion by bacteria. When a new container is, therefore, connected to the tube, it is necessary to sterilize the connector portion. Specifically, the abdominal cavity has not the ability of defending against bacteria at all and it is feared that the peritonitis is caused when bacteria enter into the abdominal cavity and propagate themselves. Accordingly, when connecting the tube, it is required that the connector portion is preferably sterilized to prevent bacteria from entering to the cavity.

However, although the disinfection using the liquid chemicals mentioned above is relatively simple, there exist resistant bacteria against the liquid chemicals and if there is an organism, the disinfection effect is reduced, furthermore, there is a side effect affecting the human body. Accordingly, it is necessary to perfectly prevent bacteria from entering to the cavity.

Recently, a thermal sterilization method has been studied as a solution to such problems. However, a steam sterilization which is one of the thermal sterilization also requires a large equipment in the same manner as the radioactive sterilization. Further, although a flame sterilization using an alcohol lamp or the like has been used until now, the flame sterilization is in danger such as a burn, a fire or the like and the sterilization does not make completely if the operator is not skilled to a certain degree.

EP—A—0116921 is a European Patent Specification published after the filing date of this application and based on a European Patent Application filed after the filing date of this application but claiming an earlier priority date. That specification shows a microwave sterilizer in which the microwave electric field generates heat within a liquid and that heat is used to sterilize some device. This document is only relevant to the extent permitted by Articles 54 (3) and 56 of the European Patent Convention.

FR—A—2305101 discloses a sterilizing apparatus which generates an electric field used to sterilize a liquid.

GB—A—1441701 discloses an apparatus for the heat sterilization of a device and the apparatus comprises a high frequency electrical source which generates a high frequency current. A coil is connected to the high frequency electrical source to generate a magnetic field in response to the high frequency current supplied thereto. A sterilization chamber is provided for receiving the device to be sterilized and around which the coil is wound. The apparatus disclosed in FR—A—2493682 also operates by generating a magnetic field in response to a high frequency current.

It is an object of the present invention to provide a sterilization apparatus suitable for receiving a tubular connector of devices to be sterilized so that it is effectively sterilized.

According to this invention, there is provided apparatus for the heat sterilization of a device, the apparatus comprising a high frequency electrical source which generates a high frequency current, a coil connected to the high frequency electrical source to generate a magnetic field in response to the high frequency current supplied thereto, and a sterilization chamber for receiving the device to be sterilized and around which the coil is wound, characterized in that the coil is shaped generally as an elongated ring bent substantially through a right angle so that the coil has an L-shaped configuration, in that the coil is wound on a former which defines said L-shaped form for the coil, and in that said sterilization chamber is formed inside one of the two end portions of the L-shaped former between two opposite sides of the former, said two opposite sides at the one end portion being spaced wider apart than the two opposite sides at the other end portion of the L-shaped former.

2

The device to be sterilized may be a tubular connector, of which only a portion to be sterilized is formed of a conductor.

An embodiment of the present invention will now be described with reference to the accompanying drawings, in which:

Fig. 1 is a perspective view of a commonly known sterilization apparatus;

Figs. 2(A) and 2(B) are a perspective view and a cross-sectional view showing an example of a device to be sterilized by the sterilization apparatus of the present invention respectively;

Figs. 3 and 4 are cross-sectional views of tubular connectors to be sterilized by the sterilization apparatus of the present invention, respectively;

Fig. 5 is a side view showing an aspect of the coil in use; and

Fig. 6 is a perspective view showing a coil according to the present invention used in the sterilization apparatus.

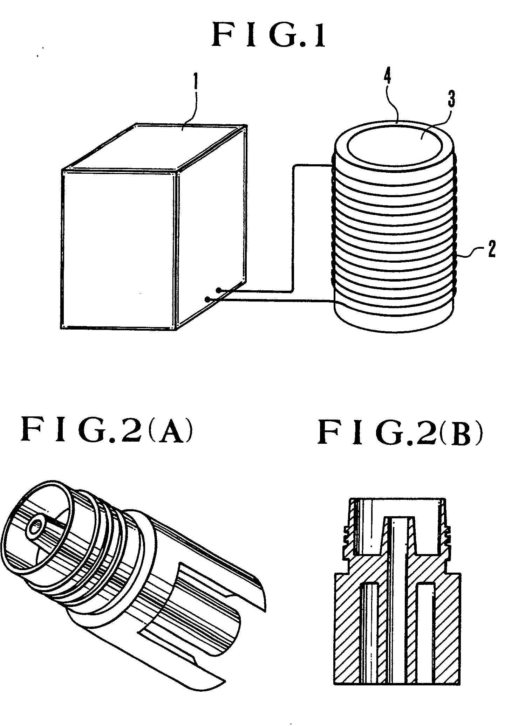

Referring to Fig. 1, a generally known sterilization apparatus includes a high-frequency current generating source 1 for generating a high-frequency current. A coil 2 is wound on the outside periphery of a cylindrical member 4 forming a sterilization chamber 3. The coil 2 is connected to the high-frequency current generating source 1 and generates a magnetic field due to the high-frequency current supplied from the source 1.

In order to sterilize a medical device by means of the sterilization apparatus constructed above, the medical device is entered in the sterilization chamber 3 together with a conductor and the high-frequency current source 1 is operated to supply the high-frequency current to the coil 2. An eddy current is generated in the conductor by the electromagnetic induction action due to the magnetic field generated by the coil 2. The conductor is heated by the Joule heat due to the eddy current loss to become a high temperature. Consequently, the medical device inserted in the chamber together with the conductor and disposed adjacent to the conductor also becomes a high temperature, so that the device to which bacteria are attached can be sterilized perfectly. In this case, the medical device is formed of an insulator. However, if the medical device is formed of a conductor, it is not necessary to insert the conductor into the chamber 3 together with the device but only the medical device may be entered into the chamber 3. In this case, an eddy current is generated in the conductor forming the medical device so that the conductor is heated to be a high temperature and the medical device is thus sterilized. In the case where the medical device is formed of a conductor, only a desired portion thereof, that is, a portion required to be sterilized may be formed of a conductor so that only the desired portion can be selectively sterilized.

The conductor is desirably formed of iron, nickel, cobalt, or an alloy including such metals and has preferably a superior characteristic of resisting a high temperature, not oxidized and not deteriorated at a high temperature. In order to completely sterilize medical devices, it is desirable to heat the medical devices at a temperature of 200°C or more in a predetermined time. In order to satisfy such a heating condition, it is considered that the magnetic flux density of the high frequency magnetic field of 10 gausses or more is proper. This value is dependent on a material, a shape, a size and the like of the medical device to be sterilized and can be selected to be suitable for each device to be sterilized. The high-frequency current generating source 1 described above may be one which is used in a known high-frequency induction heating device and the performance of the source can be determined from the sterilization temperature and time on the basis of the flux density, the material, the shape and the size of the device in association with the inductance of the coil 2. The cylindrical member 4 forming the chamber 3 is formed with at least one open end and formed of a non-conductive, heat-resisting material such as ceramic or heat-resisting plastic. Although the chamber 3 may be formed on the peripheral portion of the coil 2 instead of being formed within the coil, a large loss is suffered with regard to the heat efficiency.

One example where a device was sterilized by using the sterilization apparatus is now described.

## Example

An output power and a frequency of the high-frequency current generating source 1 were 135 watts and 10 KHz, respectively, and the magnetic flux density of the coil 2 at this time was maximum 500 gausses. A tubular connector, as shown in Figs. 2(A) and 2(B), formed of ferritic stainless steel and which is actually used in the peritoneal dialysis was used as a device to be sterilized. In order to confirm the sterilization ability, the indicator bacteria of $10^5$ were attached on the inner side of the connector, and then the connector was set in the chamber 3. The time required to sterilize all the indicator bacteria to death was measured. This measured value was compared with times required to sterilize all the indicator bacteria to death by means of a flame sterilization and liquid chemicals disinfection using an undiluted solution of ISODINE®, respectively. In this case, as the indicator bacteria, the spore of B. Stearothermophillus was used for a most heat-resisting bacteria and the spore of B. Subtilis was used for a bacteria which can be extremely resisted against a disinfectant. The following table shows the experimental results.

TABLE

|  | Time Required To Sterilize All The Indicator Bacteria | |
| --- | --- | --- |
|  | B. Stearoth-ermophillus | B. Subtilis |
| Sterilization by high-frequency induction heating sterilization apparatus | 10 seconds | 10 seconds or less |
| Flame Sterilization | 15 seconds | 15 seconds |
| Disinfection Using Liquid Chemicals | Not sterilized in 30 seconds | Not sterilized in 30 seconds |

As seen from this table, the known high-frequency induction heating sterilization is rapidly and completely effected as compared with the flame sterilization and the sterilization using liquid chemicals. Furthermore, there is no danger such as a fire and a burn in the flame sterilization, and a patient can easily use it by oneself. The sterilization apparatus and the device can be also used repeatedly. In this repeated use, the high-frequency current generating source 1 may be provided with a timer unit and a current limitation circuit to maintain the sterilization time and the current constant so that the sterilization treatment can be uniformly and completely performed. Further, as described above, since only the portion necessary to be sterilized in the device can be formed of a conductor to selectively sterilize only the portion, a portion for which the sterilization treatment is structurally difficult by means of the flame sterilization or the sterilization using liquid chemicals can be extremely easily sterilized by such a commonly known heat sterilization apparatus.

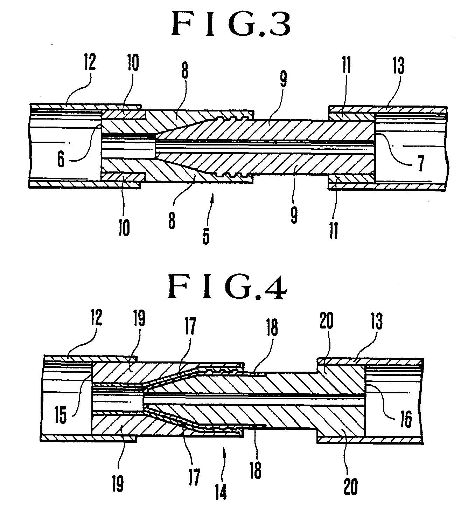

Figs. 3 and 4 show medical tubular connectors 5 and 14 which can be sterilized by the high frequency induction of such an apparatus. The connector 5 shown in Fig. 3 is consisted of a female connector 6 and a male connector 7 most of which are formed of conductors 8 and 9, respectively, and only portions coupled to tubes 12 and 13 are formed of heat-insulating materials 10 and 11. The connector 14 is consisted of a female connector 15 and a male connector 16 of which only surfaces coming in contact with each other are formed of conductors 17 and 18 and most of which are formed of heat-insulating materials 19 and 20 to which tubes 12 and 13 are connected, respectively.

The tubular connectors 5 and 14 constructed above contain the conductors 8, 9, 17 and 18 in which an eddy current is generated when the connectors 5 and 14 are disposed in the sterilization chamber 3 to receive the magnetic field generated from the coil 2, so that the conductors can be heated to sterilize the connectors. Although the connector includes relatively complicated connecting portion and it is difficult to sterilize the inside portion of the female connector, even such a portion can be completely and quickly sterilized by this sterilization method if the portion is formed of a conductor at least.

In Figs. 3 and 4, there are shown the connectors coupled to each other. The sterilization treatment of the connector may be made just before or after coupling the connector. The bonding between the conductor and the heat-insulating material in the connector is made by methods, for example, of forcedly fitting the conductor into the heat-insulating material, using an adhesive, welding the conductor to the material, or the like in the case of the structure shown in Fig. 3, while it is made by, for example, a plating, an evaporation, or a coating in the case of the structure shown in Fig. 4. The above description was made to the case where both the female and male connectors are sterilized. However, if only one of the connectors is repeatedly used and the other is used only one time, only one connector used repeatedly may be formed of a conductor as described above. The connection between a female connector and a male connector can be made, for example, by screws, by fitting projections to grooves, by fittingly inserting one to the other, by attracting one to the other by means of a magnet, or the like, and a mechanism for locking the connection portion may be provided in the connector.

In Figs. 3 and 4, if the tube is not formed of a heat-resisting material, it is desirable that the portion of the connector which is connected to the tube is formed of a heat-resisting, heat-insulating material such as ceramic, glass, heat-resisting plastic, silicone rubber and cork.

The tubular connector is suitable for the connection of the tube for coupling the abdominal cavity to the dialysate container in the above-mentioned peritoneal dialysis, specifically the continuous ambulatory peritoneal dialysis and which is repeatedly used while exchanging the container and is useful for the repeated use by sterilizing it by means of a sterilization apparatus of the present invention as defined hereinafter.

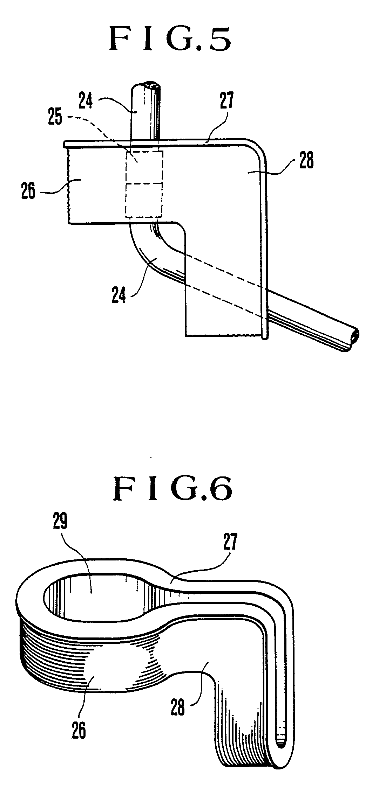

A coil 26 shown in Figs. 5 and 6 is bent to be L-shaped. In detail, the coil 26 is wound on a supporting member 27 and contains a sterilization chamber 29 which is widely formed in one end extending from a bent portion 28 to left horizontally. As described above, in the peritoneal dialysis, a straight tube or a tubular connector is used to connect the abdominal cavity with the dialysate container. Furthermore, a tube or a tubular connector is used for the transfusion and the infusion. Since such a tube or a tubular connector is straight, it is relatively difficult to set it in a sterilization chamber 3 around which a cylindrical coil 2 is wound as shown in Fig. 1. In order to solve this problem, the coil 26 shown in Figs. 5 and 6 is bent at its middle portion so that a connector 25 coupling between tubes 24 is easily set within the chamber 29 within the coil 26 as shown in Fig. 5. The connector 25 can be surely prevented from being contaminated after sterilization by sterilizing the connector in the coupling state as compared with the case where the connector is coupled after sterilization.

In the case of the coil 26 shown in Fig. 5, since the magnetic field is rather weak in the vicinity of the bent portion 28 of the coil 26, it is desirable that the device such as the connector is disposed at a portion other than the bent portion as shown in Fig. 5. The bent portion 28 may be gradually bent over a wide range or sharply bent in a narrow range. Although the bent angle is not limited to a specific angle, it is desirable that the angle of about 90°, that is, the L-shaped angle is effective for sterilization.

## Claims

1. Apparatus for the heat sterilization of a device, the apparatus comprising a high frequency electrical source (1) which generates a high frequency current, a coil (26) connected to the high frequency electrical source (1) to generate a magnetic field in response to the high frequency current supplied thereto, and a sterilization chamber (29) for receiving the device to be sterilized and around which the coil (26) is wound, characterized in that the coil (26) is shaped generally as an elongated ring bent substantially through a right angle so that the coil has an L-shaped configuration, in that the coil is wound on a former (27) which defines said L-shaped form for the coil (26), and in that said sterilization chamber (29) is formed inside one of the two end portions of the L-shaped former (27) between two opposite sides of the former, said two opposite sides at the one end portion being spaced wider apart than the two opposite sides at the other end portion of the L-shaped former.

2. Apparatus according to claim 1, characterized by means disposed in the vicinity of the device in said sterilization chamber (29) in use of the apparatus, said means serving to generate an eddy current when it is exposed to the magnetic field to thereby produce heat so that the device is sterilized by the heat thereby produced.

3. Apparatus according to claim 1, characterized in that the device is formed substantially or partly of conductive material and in that the device is sterilized by heat produced by eddy current generated in the conductive material in response to exposure to the magnetic field produced by the coil (26).

4. Apparatus according to any of claims 1 to 3 characterized in that the device is a tubular connector (25).

5. Apparatus according to claim 4, characterized in that the tubular connector (25) is a connector used in the continuous ambulatory peritoneal dialysis.

## Patentansprüche

1. Vorrichtung zur Sterilisierung eines Gerätes durch Wärme, mit einer elektrischen Hochfrequenzquelle (1), die einen Hochfrequenzstrom erzeugt, einer an die elektrische Hochfrequenzquelle (1) angeschlossenen Spule (26) zur Erzeugung eines Magnetfeldes in Abhängigkeit von dem zugeleiteten Hochfrequenzstrom, und mit einer Sterilisationskammer (29) zur Aufnahme des zu sterilisierenden Gerätes, um das die Spule (26) gewickelt ist, dadurch gekennzeichnet, daß die Spule (26) allgemein als länglicher im wesentlichen rechtwinklig abgewinkelter Ring geformt ist, so daß sie eine L-förmige Gestalt aufweist, daß die Spule auf ein Formteil (27) gewickelt ist, das die L-Form für die Spule (26) festlegt, und daß die Sterilisationskammer (29) innerhalb eines der beiden Endabschnitte des L-förmigen Formteiles (27) zwischen zwei sich gegenüberliegenden Seiten des Formteiles ausgebildet ist, wobei die sich gegenüberliegenden Seiten an dem einen Endabschnitt einen größeren Abstand zueinander haben als die sich gegenüberliegenden Seiten in dem anderen Endabschnitt des L-förmigen Formteils.

2. Vorrichtung nach Anspruch 1, gekennzeichnet durch Mittel, die in der Nähe der Vorrichtung in der Sterilisationskammer (29) während des Gebrauchs der Vorrichtung angeordnet sind, wobei die Mittel zur Erzeugung eines Wirbelstromes dienen, wenn es dem Magnetfeld ausgesetzt ist, um dadurch Wärme zu erzeugen, so daß das Gerät durch die so hervorgerufene Wärme sterilisiert wird.

3. Vorrichtung nach Anspruch 1, dadurch gekennzeichnet, daß das Gerät im wesentlichen oder teilweise aus einem leitfähigen Material gebildet ist, und daß das Gerät durch die Wärme sterilisiert wird, die durch Wirbelströme in dem leitenden Material in Abhängigkeit von dem Einfluß durch das Magnetfeld erzeugt wird, das durch die Spule (26) hervorgerufen wird.

4. Vorrichtung nach einem der Ansprüche 1 bis 3, dadurch gekennzeichnet, daß das Gerät ein rohrförmiges Verbindungsstück (24) ist.

5. Vorrichtung nach Anspruch 4, dadurch gekennzeichnet, daß das rohrförmige Verbindungsstück (24) ein Verbindungsstück ist, das bei der kontinuierlichen ambulanten peritonealen Dialyse eingesetzt wird.

**Revendications**

1. Appareil pour la stérilisation à la chaleur d'un instrument, cet appareil comprenant une source (1) d'électricité à haute fréquence qui génère un courant à haute fréquence, une bobine (26) branchée à la source (1) d'électricité à haute fréquence pour créer un champ magnétique en réponse au courant à haute fréquence qui lui est fourni, et une chambre de stérilisation (29) pour recevoir l'instrument à stériliser et autour de laquelle la bobine (26) est enroulée, caractérisé en ce que la bobine (26) est conformée dans l'ensemble comme un anneau allongé incurvé substantiellement à angle droit de sorte qu'elle a une configuration en L, en ce que la bobine (26) est enroulée sur un support (27) qui définit cette forme en L de cette bobine (26), et en ce que la chambre de stérilisation (29) est formée à l'intérieur de l'une des deux parties extrêmes du support en L (27) entre deux côtés opposés de ce dernier, ces deux côtés opposés de l'une des parties extrême étant espacés plus largement que les deux côtés opposés de l'autre partie extrême du support en L.

2. Appareil selon la revendication 1, caractérisé par un moyen disposé dans la chambre de stérilisation (29), au voisinage de l'instrument en usage dans l'appareil, ce moyen servant à engendrer un courant de Foucault quand il est exposé au champ magnétique afin de produire ainsi de la chaleur pour que l'instrument soit stérilisé par la chaleur produite de cette façon.

3. Appareil selon la revendication 1 caractérisé en ce que l'instrument est composé substantiellement ou partiellement de matière conductrice et en ce que l'instrument est stérilisé par la chaleur produite par un courant de Foucault engendré dans la matière conductrice en réponse à son exposition au champ magnétique produit par la bobine (26).

4. Appareil selon l'une quelconque des revendications 1 à 3 caractérisé en ce que l'instrument est un connecteur tubulaire (25).

5. Appareil selon la revendication 4 caractérisé en ce que le connecteur tubulaire (25) est un connecteur utilisé dans la dialyse péritonéale ambulatoire continue.

# F I G.1

# F I G.2(A)

# F I G.2(B)

# FIG.3

# FIG.4

# FIG.5

# FIG.6